# EUROPEAN PATENT APPLICATION

(11) **EP 2 298 290 A1**
(43) Date of publication of application: **23.03.2011**
(21) Application number: 09170405.6
(22) Date of filing: 16.09.2009
(51) Int. Cl.: A61K 9/28, A61K 9/20

(54) **Controlled release composition comprising levetiracetam**

(71) Applicant: LEK Pharmaceuticals d.d., 1526 Ljubljana (SI)
(72) Inventor: The designation of the inventor has not yet been filed

(57) **Abstract**

The present invention relates to a controlled release pharmaceutical composition comprising levetiracetam.

## Description

### Field of the Invention

The present invention relates to a controlled release pharmaceutical composition comprising levetiracetam.

### Description of the background art

Levetiracetam is disclosed as a protective agent for the treatment and the prevention of hypoxic and ischemic type aggressions of the central nervous system in the European patent No. EP 0 162 036 A and has the following formula. 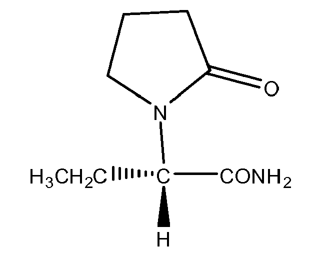 The chemical name of levetiracetam, a single enantiomer, is (-)-(S)-a-ethyl-2-oxo-1-pyrrolidine acetamide, its molecular formula is C₈Hₗ₄N₂O₂ and its molecular weight is 170.21.

Levetiracetam is marketed under the registered trademark Keppra®. Levetiracetam is available as 250 mg, 500 mg, 750 mg and 1000 mg tablets and as a clear, colorless, grape-flavored liquid (100 mg/ml) for oral administration. Recently, an extended release formulation has been made available under the trade name KEPPRA XR^{™}, which contains 500 mg or 750 mg of levetiracetam.

Levetiracetam has a relatively low order of toxicity and a relatively high therapeutic index. The twice daily dosing regimen for immediate-release levetiracetam tablets is well tolerated but with few incidences of neuropsychiatric adverse events like somnolence, fatigue, coordination difficulties and behavioral abnormalities. These adverse events are proportionate to the drug plasma level. The prior art records few documents describing controlled release levetiracetam formulations.

WO 2001/51033 provides for a solid pharmaceutical compound that can be administered orally, permitting controlled release of at least one active substance which can be Levetiracetam consisting of a homogeneous mixture comprising active substance, at least one matrix excipient between 5 and 95% by weight; at least one entero-soluble polymer between 2 and 50% by weight and at least one alkalinizing agent soluble in a aqueous phase under conditions of physiological pH, of at least 0.5 to 50% by weight in relation to the total weight of the compound.

WO 2003/101428 provides a pharmaceutical composition, which has a delayed active substance (which can be Levetiracetam) release and can be obtained by means of a special compacting method for which organic solvents and water are not required. Said pharmaceutical composition preferably exists in the form of individual active substance compartments or breaks down into compartments of this type when brought into contact with aqueous media.

PCT application WO 2006/088864 discloses a controlled release formulation of levetiracetam, which comprises an immediate release component and a modified release component, such that the immediate release component comprises a first population of levetiracetam and the modified release component preferably comprises a second population of levetiracetam and a controlled release constituent

The PCT application WO 2006/080029 discloses an extended release tablet with the core containing levetiracetam and water dispersible rate controlling polymer and further teaches that the tablet core is optionally coated with a combination of water non-dispersible and/or water dispersible polymer.

WO 2006/123357 exemplifies once-daily oral matrix based extended release pharmaceutical compositions of levetiracetam, comprising a rate-controlling agent which is optionally coated using a coating selected from (i) an active ingredient permeable coating surrounding the unit dosage form, and (ii) an active ingredient impermeable coating covering one or more surfaces but not all the surfaces of the unit dosage form.

Another PCT application WO2008/062446 relates to extended release pharmaceutical compositions of Levetiracetam, which exhibits no adverse food effect. The extended release tablet of Levetiracetam is with a core comprising of Levetiracetam and water dispersible rate controlling polymer, and the tablet core is optionally functional coated comprising a combination of water non-dispersible and/or water dispersible polymer. It provides extended therapeutically effective plasma levels over a twenty four hour period with diminished incidences of neuropsychiatric adverse events by eliminating the troughs and peaks of drug concentration in a patient's blood plasma.

WO2008/006528 describes a controlled release pharmaceutical formulation comprising levetiracetam and at least one hydrophilic matrix agent or a combination of hydrophilic and lipophilic or inert matrix agents providing controlled release.

WO2008/113901 describes controlled release formulation of levetiracetam comprising a core of levetiracetam, water soluble polymer and a lubricant, and outer water semi-permeable layer

WO2009/069089 is concerned with a controlled release pharmaceutical composition which comprises an immediate release core comprising of levetiracetam; and a release rate-controlling membrane coating of hydrophobic polymer(s).

WO2009/087675 describes an extended release pharmaceutical composition comprising levetiracetam comprising levetiracetam wherein the core is coated with a rate controlling composition. Said rate controlling composition comprises one or more hydrophilic agents and one or more hydrophobic agents.

There is however still a need in the art for levetiracetam controlled release formulation with improved properties.

The object of the present invention is to provide a robust controlled release pharmaceutical composition comprising levetiracetam.

### Summary of the invention

The aspects, advantageous features and preferred embodiments of the present invention summarized in the following items, respectively alone or in combination, further contribute to solving the object of the invention:
1) A controlled release pharmaceutical composition, comprising levetiracetam and at least one lipophilic matrix agent.
2) The controlled release pharmaceutical composition according to item (1) comprising from 10 to 60 % by weight of at least one lipophilic matrix agent
3) The controlled release pharmaceutical composition according to item (1), comprising from 30 to 40 % by weight of at least one lipophilic matrix agent
4) The controlled release pharmaceutical composition according to any one of items (1) - (3), wherein lipophilic matrix agent is selected from the group consisting of waxes, fatty acids, fatty alcohols, fatty acids esters, glycerides and mixtures thereof.
5) The controlled release pharmaceutical composition according to item (4), wherein said lipophilic matrix agent is hydrogenated castor oil and/or glyceryl behenate
6) The controlled release pharmaceutical composition according to any one of items (1) - (5), further comprising a lubricant
7) The controlled release pharmaceutical composition according to item (6), comprising from 0.1 to 4 % by weight of a lubricant, preferably from 0.5 to 3 % by weight of a lubricant
8) The controlled release pharmaceutical composition according to any one of items (6) - (7), wherein said lubricant is selected from the group consisting of hydrogenated vegetable oil, glyceryl behenate, polyethylene glycol, magnesium stearate and sodium stearyl fumarate, wherein said hydrogenated vegetable oil is preferably hydrogenated castor oil
9) The controlled release pharmaceutical composition according to any one of items (1) to (8), wherein a pharmaceutical composition is in the form of a tablet.
10)The controlled release pharmaceutical composition according to item (9), wherein
   said tablet further comprises a coating.
11)The controlled release tablet comprising levetiracetam,
   from 30 % to 40 % by weight of lipophilic matrix agent, wherein said lipophilic matrix agent is hydrogenated castor oil and/or glyceryl behenate, and
   from 0.1 % to 4 % by weight of lubricant, wherein said lubricant is selected from the group consisting of hydrogenated castor oil, polyethylene glycol, glyceryl behenate magnesium stearate and sodium stearyl fumarate
12)The controlled release tablet comprising
   from 55 % to 70 % by weight of levetiracetam,
   from 30 % to 40 % by weight of lipophilic matrix agent, wherein said lipophilic matrix agent is hydrogenated castor oil and/or glyceryl behenate, and from 0.1 % to 4 % by weight of lubricant, wherein said lubricant is selected from the group consisting of hydrogenated castor oil, polyethylene glycol, glyceryl behenate magnesium stearate and sodium stearyl fumarate

### Description of further Advantages and Preferred Embodiments of the Invention:

In the following, the present invention will be described in more detail by preferred embodiments and examples while referring to the attached drawings, noting, however, that these embodiments, examples and drawings are presented for illustrative purposes only and shall not limit the invention in any way

The term "controlled release pharmaceutical composition", as referred to herein, is defined to mean oral pharmaceutical compositions which when administered releases the active medicament at a relatively constant rate and provide plasma concentrations of the active medicament that remain substantially invariant with time within the therapeutic range of the active medicament over a 24-hour period and encompasses "prolonged release", "extended release", "modified release", "delayed release" and "sustained release" compositions. The compositions according to the present invention deliver a therapeutically effective amount of levetiracetam to a patient for 24 hours following a once-daily administration. The term "therapeutically effective amount" intends to describe an amount of the active agent which stops or reduces the progress of the condition to be treated or which otherwise completely or partly cures or acts palliatively on the condition.

The term by weight, as referred to herein, is defined to mean with respect to total weight of pharmaceutical composition in question.
Fig. 1 shows cumulative amount of drug released (%) from pharmaceutical formulation obtained in Example 1 plotted versus time (h); and analyzed under test 1, 2 and 3 conditions.
Fig. 2 shows cumulative amount of drug released (%) from pharmaceutical formulation described in Comparative example 1 plotted versus time (h); and analyzed under tests 1, 2 and 3 conditions.
Fig. 3 shows cumulative amount of drug released (%) from pharmaceutical formulations obtained in Example 1 and Comparative example 2 plotted versus time (h); and analyzed under test 4 conditions.

Controlled drug delivery systems frequently offer significant benefit in comparison with immediate release dosage forms from the efficiency and safety viewpoint. They enable maintaining a desired blood level of a drug over a longer period of time resulting in increased patient compliance by reducing the number of daily administrations and consequently less fluctuation in drug plasma concentration, all together contributing to reduced side effects and improved efficacy of the therapy. Oral controlled release formulation faces substantial changes in gastrointestinal conditions during its passage along gastrointestinal tract, *i.e.* change in pH and composition of gastrointestinal fluids (ionic strength, presence / absence of food, presence / absence of other liquids, solvents (alcohol), surfactants...), change in mechanical exposure (abrasion) due to change in gastrointestinal motility and MMC (Migrating Myoelectrical Complex). The conditions in gastrointestinal tract are subjected to high intra subject and even more inter subject variability. Additionally, various disease states like Crohn's disease, alcoholism, microbiological infections dramatically influence the conditions in gastrointestinal tract. Considering this facts, controlled release formulation with robust drug release profile which is not susceptible to changes in gastrointestinal tract is required to provide a desired release profile that assures optimal therapeutic efficacy of a drug. If the drug release profile from the formulation is subjected to changes in gastrointestinal conditions the drug release can be faster (dose dumping) or slower than intended. In both cases, severe consequences for a patient can occur including reduced therapeutic efficacy due to too low drug concentration or toxic effect due to overdosing.

The presence of alcohol may have a profound influence on release of active ingredient, *e.g.* slower release of levetiracetam can result in lower therapeutic efficacy what is of special importance in the treatment of alcohol dependence and in treatment of patients with epilepsy which abuse alcohol more frequently. Faster release of levetiracetam on the other hand might increase a risk of toxic effect due to overdosing.

Recently, levetiracetam was tested for the treatment of alcohol dependence and co-occurring anxiety disorder. It was shown that levetiracetam can be effective in alcohol dependence treatment (The American Journal of Drug and Alcohol Abuse, 34: 683-691, 2008, The American Journal of Drug and Alcohol Abuse, 34: 441-447, 2008. From safety and efficacy viewpoint it is therefore crucial to develop a controlled release formulation comprising levetiracetam characterized by a robust drug release profile independent of changes in gastrointestinal conditions and presence of alcohol.

The invention thus provides an easy to make robust controlled release pharmaceutical formulation for oral administration comprising levetiracetam as active ingredient and lipophilic matrix agent enabling controlled release. The release of levetiracetam from matrix tablet formulation is insensitive to various *in vivo* relevant dissolution testing media as well as different hydrodynamic conditions. In particular, a controlled release pharmaceutical formulation according to present invention shows excellent robustness regarding the influence of alcohol. The levetiracetam release profile of controlled release pharmaceutical formulation according to present invention is not affected by the presence of alcohol.

The pharmaceutical composition of the present invention comprises levetiracetam and at least one lipophilic matrix agent.

Preferably, the pharmaceutical composition of the present invention comprises from 45 to 90 % by weight of levetiracetam; more preferably from 50 to 80 % by weight of levetiracetam and most preferably from 55 to 70 % by weight of levetiracetam or a pharmaceutically acceptable salt thereof.

Lipophilic matrix agents provide several advantages, ranging from good stability at varying pH values and moisture levels to safe applications. Use of lipophilic matrix agents is particularly advantageous for drugs with high water solubility (such as levetiracetam) as restrictions of controlled drug release due to rapid diffusion of the dissolved drug through the hydrophilic gel network, that might occur when hydrophilic matrix system is used, is avoided.

Suitable lipophilic matrix agents include, but are not limited to waxes (such as for example white wax, bees wax, carnauba wax and the like), fatty acids (such as for example stearic, palmitic, lauric acid and the like), fatty alcohols (such as for example cetyl, cetostearyl, stearyl alcohol and the like), fatty acids esters (such as for example monostearates of propylene glycol, monostearates of sucrose, sucrose distearate and the like), glycerides (such as for example monoglycerides, diglycerides, triglycerides: palmitin, stearin, behenic, laurin, myristin, hydrogenated vegetable oil (such as for example hydrogenated castor oil, hydrogenated cottonseed oils), glyceril behenate and the like) and mixtures thereof. Preferred lipophilic matrix agent is hydrogenated castor oil and/or glyceryl behenate.

Preferably the pharmaceutical composition according to present invention comprises from 10 to 60 % by weight of at least one lipophilic matrix agent; more preferably from 20 to 50 % by weight, and most preferably from 30 to 40 % by weight of at least one lipophilic matrix agent.

The pharmaceutical composition of the present invention might further comprise a lubricant. Various suitable lubricants include but are not limited to stearic acid, talc, hydrogenated vegetable oil (*e.g.* hydrogenated castor oil), sodium lauryl sulphate, glyceryl behenate, polyethylene glycol, magnesium stearate and sodium stearyl fumarate. Preferably lubricant is selected from the group consisting of hydrogenated castor oil, polyethylene glycol, glyceryl behenate magnesium stearate and sodium stearyl fumarate, more preferably lubricant is magnesium stearate and / or hydrogenated castor oil.

Preferably pharmaceutical composition of the present invention comprises from 0.1 to 4 % by weight of a lubricant, more preferably from 0.5 to 3 % by weight of a lubricant.

In preferred embodiment the same excipient material can be used as lipophilic matrix agent and as a lubricant (*e.g.* hydrogenated castor oil, glyceryl behenate), making the pharmaceutical composition of the present invention more convenient and appropriate for industrial production.

The pharmaceutical composition according to present invention may optionally further comprise other excipients which are commonly used in the art and known to any person skilled in the art. These include, but are not limited to binders, fillers glidants, antiadhesive agents and the like.

Various useful binders include but are not limited to hydroxypropylcellulose (Klucel™-LF), hydroxypropyl methylcellulose (Methocel™), polyvinylpyrrolidone (PVP-K25, PVP-K29, PVP-K30), powdered acacia, gelatin, guar gum, carbomer (*e.g.* carbopol), methylcellulose, polymethacrylates, and starch.

Various useful fillers include but are not limited to starches, lactose, mannitol, cellulose derivatives and the like. Different grades of lactose include but are not limited to lactose monohydrate and lactose anhydrous. Different grades of starches included but are not limited to maize starch, potato starch, rice starch, wheat starch, pregelatinized starch, fully pregelatinized starch. Different cellulose compounds that can be used include microcrystalline cellulose and powdered cellulose. Examples of microcrystalline cellulose products include but are not limited to Avicel™ PH 101, PH102, PH301, PH302 and PH-F20, microcrystalline cellulose 114, and microcrystalline cellulose 112.

Various useful glidants include but are not limited to starches, colloidal silicon dioxide and talc.

Various useful antiadhesive agents include but are not limited to colloidal silicon dioxide and talc.

A pharmaceutical compositions according to the present invention is preferably in solid form, including tablets, capsules, caplets, lozenges and sachets.
Tablets may be suitably coated (film coated tablets, pills). Capsule formulations may cover both soft and hard capsules. A pharmaceutical compositions according to the present invention is preferably in the form of tablet, more preferably coated tablet with appropriate film coating material.

The coating can be selected from the group of ready to form dispersion such as OPADRY. The coating dispersion comprises hydrophilic film forming polymer (such as for example low viscosity HPMC, HPC, PVA (polyvinylalcohol) and the like), plastificators (*e.g.* PEG), colorants and may optionally include other excipients such as antitacking agents

Any method for film coating, known in the field of the pharmaceutical technology, may be used.

A levetiracetam pharmaceutical composition according to present invention can be prepared by processes well known to those skilled in the art. For example, levetiracetam tablets according to present invention can be prepared by wet granulation, dry granulation, melt granulation, direct compression and the like.

Preferably, the core of the tablets of the present invention is formulated with levetiracetam, at least one lipophilic matrix agent and a lubricant. More preferably the core of the tablets of the present invention is formulated with levetiracetam, hydrogenated castor oil and / or glyceryl behenate as lipophilic matrix agent and magnesium stearate, hydrogenated castor oil or glyceryl behenate as lubricant.

A pharmaceutical composition of the present invention comprises preferably 500, 750 or 1000 mg of levetiracetam as the respective dose.

In one embodiment the controlled release tablet according to present invention comprises levetiracetam,
from 30 % to 40 % by weight of lipophilic matrix agent, wherein said lipophilic matrix agent is hydrogenated castor oil and/or glyceryl behenate, and from 0.1 % to 4 % of lubricant, wherein said lubricant is selected from the group consisting of hydrogenated castor oil, polyethylene glycol, glyceryl behenate magnesium stearate and sodium stearyl fumarate In another embodiment the controlled release tablet according to present invention comprises levetiracetam
from 55 % to 70 % by weight of levetiracetam,
from 30 % to 40 % by weight of lipophilic matrix agent, wherein said lipophilic matrix agent is hydrogenated castor oil and/or glyceryl behenate, and from 0.1 % to 4 % by weight of lubricant, wherein said lubricant is selected from the group consisting of hydrogenated castor oil, polyethylene glycol, glyceryl behenate magnesium stearate and sodium stearyl fumarate

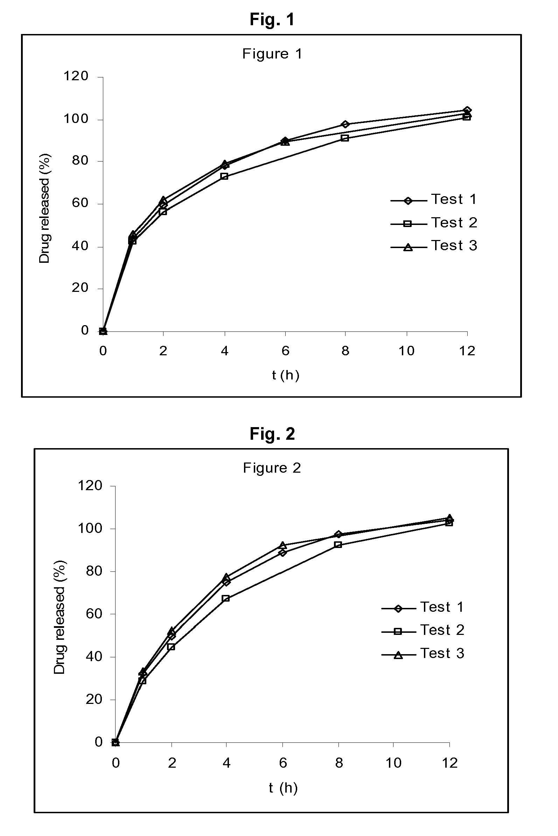

The present invention can thus provide a pharmaceutical composition that is not susceptible to ethanol. As it is evident form Table 1 and Figures 1 and 2 the difference between the amount of levetiracetam released under Test 1 conditions (simulating influence of alcohol) compared to the amount released under normal physiological conditions (Test 2) is significantly smaller for pharmaceutical formulation according to present invention compared to the composition of Comparative example 1. In other words, the amount of levetiracetam released from pharmaceutical formulation according to present invention is less affected by the presence of ethanol compared to pharmaceutical formulation described in Comparative example 1. The release of levetiracetam is slower in the presence of ethanol in the case of pharmaceutical formulation described in Comparative example 1.

Furthermore pharmaceutical composition according to present invention is very robust to the influence of mechanical forces that can occur when tablet passes the gastrointestinal tract as it can be seen from the results in Table 2 and Figure 1. Only minor differences in the release of levetiracetam are observed when the pharmaceutical composition according to present invention is subjected to test conditions that simulate the situation in gastrointestinal tract with intense mechanical stress like transfer of matrix tablet through pylorus, presence of food or MMC, compared to the results obtained under test conditions that mimic physiologically normal conditions (i.e. results under Test 3 conditions compared with results under Test 2)

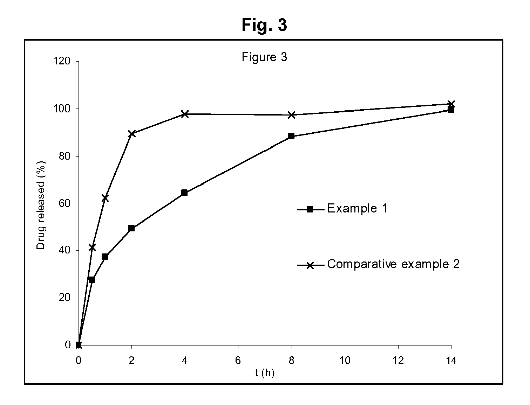

Excellent robustness regarding the influence of alcohol of the pharmaceutical composition according to present invention is further supported by the results presented in Figure 3. The pharmaceutical formulation obtained in Comparative example 2 (that contains inert matrix agent in addition to lipophilic matrix agent) shows considerable susceptibility to the presence of ethanol as it exhibits a burst release of levetiracetam. The pharmaceutical composition according to present invention, however, retains its controlled release properties when subjected to the same conditions (i.e. Test 4 conditions).

### Examples

### Example 1:

| Core | Function | mg | % |
|---|---|---|---|
| Levetiracetam | Active | 500. 00 | 62. 50 |
| Castor oil, | Lipophilic matrix | 284. 00 | 35. 50 |
| hydrogenated | agent | | |
| Castor oil, | Lubricant | 16. 000 | 2. 00 |
| hydrogenated | | | |
| | | 800. 00 | 100. 00 |

The tablets were prepared by wet granulation where levetiracetam is granulated with dispersion of hydrogenated castor oil in acetone.

Preparation of granulation liquid: hydrogenated castor oil is dispersed in acetone and the dispersion is mixed on a magnetic stirrer for 4h.

Levetiracetam, previously sieved, is granulated with acetone dispersion. The granulate is dried in vacuum dryer and afterwards sieved through a proper sieve. Finally, hydrogenated castor oil is added to dry and sieved granulate and homogenously blended and compressed into 800 mg tablets.

### Example 2:

| Core | Function | mg | % |
|---|---|---|---|
| Levetiracetam | Active | 500. 00 | 62. 50 |
| Castor oil, hydrogenated | Lipophilic matrix agent | 284. 00 | 35. 50 |
| Magnesium stearate | Lubricant | 16. 000 | 2. 00 |
| | | 800. 00 | 100. 00 |

Direct compression was used as manufacturing procedure for preparation of the tablets.

Levetiracetam and hydrogenated castor oil are homogenously blended and sieved. Magnesium stearate is added to a mixture, homogenously blended and compressed into tablets, 800 mg.

### Example 3:

| Core | Function | mg | % |
|---|---|---|---|
| Levetiracetam | Active | 500. 00 | 62. 50 |
| Glyceryl behenate | Lipophilic matrix agent | 284. 00 | 35. 50 |
| Glyceryl behenate | Lubricant | 16. 000 | 2. 00 |
| | | 800. 00 | 100. 00 |

Hot melt granulation was used as manufacturing procedure for preparation of the tablets.

Glyceryl behenate is melted. Previously sieved levetiracetam is granulated with the melt. Granulate is sieved through appropriate sieve. Powdered glyceryl behenate is added to granulate, homogenously blended and compressed into tablets, 800 mg.

### Comparative example 1

Reference product KEPPRA XR 500 mg levetiracetam tablets with following qualitative composition were comparatively evaluated.

| Keppra XR | Function |
|---|---|
| Levetiracetam | Active |
| Hypromellose | Hydrophilic matrix agent |
| Polyethyleneglicol 6000 | Lubricant |
| Anhydrous colloidal silica | Glidant |
| Magnesium stearate | Lubricant |
| Polyvinyl alcohol - partially hydrolized, Titanium dioxide, Macrogol PEG 3350, Talc | Film coating |

### Comparative example 2

| Core | Function | mg | % |
|---|---|---|---|
| Levetiracetam | Active | 500.00 | 50.00 |
| Eudragit RS 30D | Inert matrix agent | 800.00 of dispersion | 24.00 |
| | | (240 mg of dry polymer) | |
| Dibutyl sebacate | Plastificator | 48.00 | 4.80 |
| Castor oil, hydrogenated | Lipophilic matrix agent | 197.000 | 19.70 |
| Anhydrous colloidal silica | Glidant | 5.000 | 0.50 |
| Magnesium stearate | Lubricant | 10.000 | 1.00 |
| | | 1000.00 | 100.00 |

Fluid bed granulation was selected as manufacturing procedure for preparation of the tablets.

Dispersion of Eudragit RS 30D and dibutyl sebacate are mixed for 1h and afterwards sprayed on levetiracetam in fluid bed granulator. Dry granulate is sieved through appropriate sieve. Hydrogenated castor oil and anhydrous colloidal silica are added to granulate, the mixture is sieved and homogenized. Magnesium stearate is added to a mixture, homogenously blended and compressed into tablets, 1000 mg.

### Dissolution tests:

Tablets of present invention as well as tablets from Comparative examples 1, 2 were subjected to dissolution tests that simulate different conditions in human gastrointestinal tract. The solubility of levetiracetam is good, and it is pH independent, therefore in particular extreme conditions that can occur in gastrointestinal tract can affect the release of levetiracetam from tablets. Four different tests were chosen for evaluation of levetiracetam extended release matrix tablets to prove robustness.

**Test 1:** Apparatus 1 (basket method), 100 rpm in 43 % ethanol media; sampling times: 1, 2, 4, 8, 12 h. Test simulates influence of alcohol on release of levetiracetam from matrix tablet after simultaneous consumption of ethanol and levetiracetam matrix tablet.

**Test 2:** Apparatus 1 (basket method), 100 rpm in potassium phosphate buffer pH 6,0; sampling times: 1, 2, 4, 6, 8, 12 h. This test mimic physiologically normal conditions under which the levetiracetam is released from the tablet.

**Test 3:** Apparatus 1 (basket method); 100 rpm, after 30 minutes tablets were transferred to plastic tubes containing 5 ml of media and 10 glass beads with density 2.5 g/cm³ and diameter 10 mm. The tablets were then shaken for 10 minutes on laboratory shaker in 300 rotations per minute. After this manipulation, tablets were transferred back to baskets and dissolution test continued. Media used was water; sampling times: 30 min,1, 2, 4, 6, 12 h. This test describes situation in gastrointestinal tract with intense mechanical stress like transfer of matrix tablet through pylorus, presence of food or MMC.

**Test 4:** Apparatus 3 (reciprocating cylinder), 25 dpm, tablets tested in 43 % of alcohol media; sampling times: 30 min, 1, 2, 4, 8, 14 h.

The results are shown in Figures 1 and 2 and 3 and in Tables 1 and 2.

**Table 1: Relative difference (%) in amount of levetiracetam released for various time points between Test 2 and Test 1 conditions.**

| t(h) | Example 1 | Comparative example 1 |
|---|---|---|
| 1 | 3. 8 | 10. 4 |
| 2 | 5. 4 | 11. 1 |
| 4 | 6. 4 | 10. 7 |
| 8 | 6. 7 | 5. 3 |

**Table 2: Relative difference (%) in amount of levetiracetam released for various time points between Test 3 and Test 2 conditions.**

| t(h) | Example 1 | Comparative example 1 |
|---|---|---|
| 1 | 2. 2 | 1. 2 |
| 2 | 2. 7 | 2. 6 |
| 4 | 1. 1 | 2. 2 |
| 12 | 1. 8 | 1. 5 |

It is evident form Table 1 and Figures 1 and 2 that pharmaceutical formulation of present invention is less susceptible to ethanol (Test 1) compared to pharmaceutical formulation described in Comparative example 1 since the difference between the amount of levetiracetam released under Test 1 conditions compared to the amount released under normal physiological conditions (Test 2) is significantly smaller for pharmaceutical formulation obtained in Example 1. In other words, the amount of levetiracetam released from pharmaceutical formulation obtained in Example 1 is less affected by the presence of ethanol compared to pharmaceutical formulation described in Comparative example 1. The release of levetiracetam is slower in the presence of ethanol in the case of pharmaceutical formulation described in Comparative example 1.

Results in Table 2 and Figure1 (comparison of results under Test 3 conditions with results under normal physiological conditions (Test 2)) show that pharmaceutical formulation of present invention shows excellent robustness to the influence of mechanical forces that can occur when tablet passes the gastrointestinal tract.

The pharmaceutical formulation obtained in Comparative example 2 shows considerable susceptibility to the presence of ethanol as it is evident from Figure 3. Said formulation exhibits burst release and the controlled release property of matrix tablet is diminished which consequently leads to therapeutic efficacy and safety issues. Test 4 shows further advantage in robustness of pharmaceutical composition of present invention.

## Claims

1. A controlled release pharmaceutical composition, comprising levetiracetam and at least one lipophilic matrix agent.

2. The controlled release pharmaceutical composition according to claim 1 comprising from 10 to 60 % by weight of at least one lipophilic matrix agent

3. The controlled release pharmaceutical composition according to claim 1, comprising from 30 to 40 % by weight of at least one lipophilic matrix agent

4. The controlled release pharmaceutical composition according to any one of claims 1 - 3, wherein lipophilic matrix agent is selected from the group consisting of waxes, fatty acids, fatty alcohols, fatty acids esters, glycerides and mixtures thereof

5. The controlled release pharmaceutical composition according to claim 4, wherein said lipophilic matrix agent is hydrogenated castor oil and/or glyceryl behenate

6. The controlled release pharmaceutical composition according to any one of claims 1 - 5, further comprising a lubricant

7. The controlled release pharmaceutical composition according to claim 6, comprising from 0.1 to 4 % by weight of a lubricant

8. The controlled release pharmaceutical composition according to any one of claims 6 - 7, wherein said lubricant is selected from the group consisting of hydrogenated castor oil, polyethylene glycol, glyceryl behenate magnesium stearate and sodium stearyl fumarate

9. The controlled release pharmaceutical composition according to any one of
claims 1 to 8, wherein a pharmaceutical composition is in the form of a tablet.

10. The controlled release pharmaceutical composition according to claim 9, wherein
said tablet further comprises a coating.

11. The controlled release tablet comprising levetiracetam,
from 30 % to 40 % by weight of lipophilic matrix agent, wherein said lipophilic matrix agent is hydrogenated castor oil and/or glyceryl behenate and
from 0.1 % to 4 % by weight of lubricant, wherein said lubricant is selected from the group consisting of hydrogenated castor oil, polyethylene glycol, glyceryl behenate magnesium stearate and sodium stearyl fumarate

12. The controlled release tablet comprising
from 55 % to 70 % by weight of levetiracetam,
from 30 % to 40 % by weight of lipophilic matrix agent, wherein said lipophilic matrix agent is hydrogenated castor oil and/or glyceryl behenate and
from 0.1 % to 4 % by weight of lubricant, wherein said lubricant is selected from the group consisting of hydrogenated castor oil, polyethylene glycol, glyceryl behenate magnesium stearate and sodium stearyl fumarate
